(19) Europäisches Patentamt
European Patent Office
Office européen des brevets

(11) Veröffentlichungsnummer: **0 378 755 B1**

# (12) EUROPÄISCHE PATENTSCHRIFT

(45) Veröffentlichungstag der Patentschrift: **29.12.93**

(51) Int. Cl.[5]: **C07D 213/30**, C07D 213/32, C07D 307/12, C07D 307/10, C07D 333/16, C07D 333/18, C07D 231/12, C07D 233/22, C07D 261/08, C07D 263/14, C07D 275/02

(21) Anmeldenummer: **89119384.9**

(22) Anmeldetag: **19.10.89**

Die Akte enthält technische Angaben, die nach dem Eingang der Anmeldung eingereicht wurden und die nicht in dieser Patentschrift enthalten sind.

## (54) Heterocyclisch substituierte alpha-Aryl-acrylsäuremethylester und ihre Verwendung.

(30) Priorität: **27.10.88 DE 3836581**

(43) Veröffentlichungstag der Anmeldung:
**25.07.90 Patentblatt 90/30**

(45) Bekanntmachung des Hinweises auf die Patenterteilung:
**29.12.93 Patentblatt 93/52**

(84) Benannte Vertragsstaaten:
**AT BE CH DE ES FR GB GR IT LI NL SE**

(56) Entgegenhaltungen:
**EP-A- 0 178 826**
**EP-A- 0 242 081**
**EP-A- 0 244 077**
**EP-A- 0 260 794**
**EP-A- 0 299 694**
**DE-A- 3 519 280**
**DE-A- 3 620 860**

**COMPREHENSIVE HETEROCYCLIC CHEMISTRY, vol 6, part 4B, A. Katritzki, C.W. Rees, Pergamon Press (New York 1984), pages 29-30.**

(73) Patentinhaber: **BASF Aktiengesellschaft**
**Carl-Bosch-Strasse 38**
**D-67063 Ludwigshafen(DE)**

(72) Erfinder: **Schuetz, Franz, Dr.**
**Budapester Strasse 45**
**D-6700 Ludwigshafen(DE)**
Erfinder: **Neubauer, Hans-Jürgen, Dr.**
**Mozartstrasse 6**
**D-4400 Münster-Hiltrup(DE)**
Erfinder: **Kuekenhoehner, Thomas, Dr.**
**Seidelstrasse 2**
**D-6710 Frankenthal(DE)**
Erfinder: **Schirmer, Ulrich, Dr.**
**Berghalde 79**
**D-6900 Heidelberg(DE)**
Erfinder: **Hofmeister, Peter, Dr.**
**Bernard-Humblot-Strasse 12**
**D-6730 Neustadt(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**THE CHEMISTRY OF HETEROCYCLIC COMPOUNDS, A. Ouilico et al., Interscience, J. Wiley and Sons, (New York 1962), pages 40-46.**

Erfinder: **Kuenast, Christoph, Dr.**
**Salierstrasse 2**
**D-6701 Otterstadt(DE)**
Erfinder: **Ammermann, Eberhard, Dr.**
**Sachsenstrasse 3**
**D-6700 Ludwigshafen(DE)**
Erfinder: **Lorenz, Gisela, Dr.**
**Erlenweg 13**
**D-6730 Neustadt(DE)**
Erfinder: **Kardorff, Uwe, Dr.**
**D 3,4**
**D-6800 Mannheim 1(DE)**

## Beschreibung

Die vorliegende Erfindung betrifft wertvolle neue heterocyclisch substituierte α-Aryl-acrylsäuremethylester mit fungizider und insektizider Wirkung und Fungizide bzw. Insektizide, die diese Verbindungen enthalten.

Es ist bekannt, Acrylsäuremethylester, z.B. den α-[2-(Benzoxazol-2'-yl-oxy)-phenyl]-β-methoxy-acrylsäuremethylester (EP-256 667) als Insektizide oder Fungizide (EP-A-0 178 826, EP-A1-0 244 077) zu verwenden. Ihre insektizide Wirkung ist jedoch unbefriedigend.

Es wurde nun gefunden, daß heterocycliscn substituierte α-Aryl-acrylsäuremethylester der allgemeinen Formel

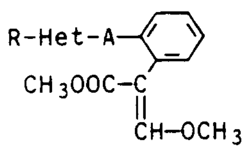

(I)

in der

R $C_1$-$C_8$-Alkyl, $C_2$-$C_8$-Alkenyl, $C_3$-$C_6$-Cycloalkyl, $C_1$-$C_4$-Alkoxy, $C_1$-$C_4$-Alkylcarbonyl, $C_1$-$C_4$-Alkoxycarbonyl oder Aryl bedeutet, wobei der aromatische Ring gegebenenfalls substituiert ist durch $C_1$-$C_8$-Alkyl, $C_3$-$C_6$-Cycloalkyl, $C_1$-$C_2$-Halogenalkyl, $C_1$-$C_4$-Alkoxy, Halogen, Cyano, Nitro,

Het einen gegebenenfalls durch Methyl an einem Stickstoffatom substituierten fünfringheteroaromatischen Rest mit 1 bis 3 Heteroatomen , ausgewählt aus der Gruppe Sauerstoff, Schwefel oder Stickstoff, der über ein C-Atom an A gebunden ist,

A Ethenylen bedeutet,

eine ausgezeichnete fungizide und insektizide Wirkung besitzen, die besser ist, als die der bekannten Acrylsäuremethylester.

Die in der allgemeinen Formel aufgeführten Reste können beispielsweise folgende Bedeutung haben:

R kann z.B. $C_1$-$C_8$-Alkyl, insbesondere $C_1$-$C_4$-Alkyl (z.B. Methyl, Ethyl, n- oder iso-Propyl, n-, iso-, sec.- oder tert.-Butyl, n-, iso-, sec.-, tert.- oder neo-Pentyl, Hexyl, Heptyl, Octyl), $C_2$-$C_8$-Alkenyl, insbesondere $C_2$-$C_3$-Alkenyl (z.B. Vinyl, Allyl, Propenyl, Isopropenyl), $C_3$-$C_6$-Cycloalkyl (z.B. Cyclopropyl, Cyclobutyl, Cyclopentyl, Cyclohexyl), $C_1$-$C_4$-Alkoxy (z.B. Methoxy, Ethoxy, n- oder iso-Propoxy, n-, iso-, sec.-oder tert.-Butoxy), $C_1$-$C_4$-Alkylcarbonyl (z.B. Acetyl, Propanoyl, Butanoyl, Pentanoyl), $C_1$-$C_4$-Alkoxycarbonyl (z.B. Methoxycarbonyl, Ethoxycarbonyl, Propoxycarbonyl, Butoxycarbonyl) oder Aryl (z.B. Phenyl) bedeuten, wobei der aromatische Ring gegebenenfalls durch einen bis drei der folgende Reste substituiert sein kann: $C_1$-$C_8$-Alkyl, insbesondere $C_1$-$C_4$-Alkyl (z.B. Methyl, Ethyl, n- oder iso-Propyl, n-, iso-, sec.- oder tert.-Butyl, n-, iso-, sec.-, tert.- oder neo-Pentyl, Hexyl, Heptyl, Octyl), $C_3$-$C_6$-Cycloalkyl (z.B. Cyclopropyl, Cyclobutyl, Cyclopentyl, Cyclohexyl), $C_1$-$C_2$-Halogenalkyl (z.B. Trifluormethyl, 2,2,2-Trifluorethyl, Pentafluorethyl, Chlormethyl, Dichlormethyl, Trichlormethyl), $C_1$-$C_4$-Alkoxy, (z.B. Methoxy, Ethoxy, Propoxy, Butoxy), Halogen (z.B. Fluor, Chlor, Brom), Cyano oder Nitro.

Het kann z.B. einen gegebenenfalls durch Methyl an einem Stickstoffatom substituierten fünfringheteroaromatischen Rest mit 1 bis 3 Heteroatomen wie Sauerstoff, Schwefel oder Stickstoff (z.B. Pyrrolyl, Furyl, Thienyl, Pyrazolyl, Imidazolyl, 1,2,4-Triazolyl, Oxazolyl, N-Methyl-pyrazolyl, Thiazolyl, Isoxazolyl, Isothiazolyl, 1,3,4-Oxadiazolyl, 1-N-Methyl-1,2,4-triazolyl, 1,3,4-Thiadiazolyl, 1,2,4-oxadiazolyl, 1,2,4-Thiadiazolyl) sein, der über ein C-Atom an A gebunden ist.

A ist Ethenylen (-CH = CH-).

Die neuen Verbindungen können beispielsweise nach folgenden Verfahren hergestellt werden:

Die Verbindungen der allgemeinen Formel I a (R und Het haben die oben angegebene Bedeutung, A = Ethenylen) und der allgemeinen Formel I b (R und Het haben die oben angegebene Bedeutung, A = Methylenoxy) erhält man aus den heterocyclisch substituierten α-Aryl-β-hydroxy-acrylsäuremethylesterderivaten der allgemeinen Formel V, die im Gleichgewicht mit den Formylderivaten VI vorliegen können, durch Umsetzung mit einem Alkylierungsmittel (z.B. Dimethylsulfat, Methyljodid) in Gegenwart einer Base (z.B. Kaliumcarbonat, Natriumcarbonat) in einem Verdünnungsmittel (z.B. Aceton). In den folgenden Formelbildern bedeutet "L" eine Abgangsgruppe (z.B. Methylsulfat, Jodid).

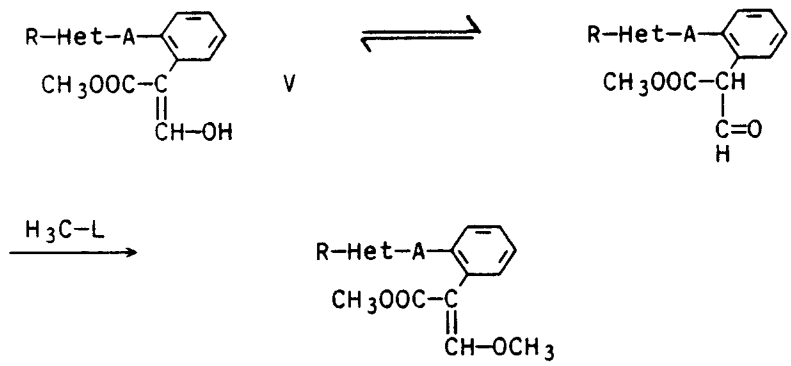


VI

Die heterocyclischen α-Aryl-β-hydroxy-acrylsäuremethylester der allgemeinen Formel V erhält man aus heterocyclisch substituierten Phenylessigsäuremethylestern der allgemeinen Formel II durch Umsetzung mit Ameisensäuremethylester unter Verwendung einer Base (z.B. Natriumhydrid, Lithiumdiisopropylamid, Natriummethanolat) in einem inerten Lösungsmittel wie z.B. Diethylether oder Tetrahydrofuran (vgl. dazu Ann. Chem. 424 (1921) 214).

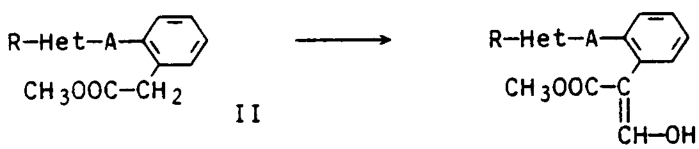

V

Die Herstellung der als Ausgangsverbindungen benötigten heterocyclisch substituierten Phenylessigsäuremethylester II a erfolgt durch Umsetzung von einem 2-Formyl-phenylessigsäuremethylester III mit einem Methanphosphonsäureester der allgemeinen Formel IV (R und Het haben die oben angegebene Bedeutung, $R^1$ = Methyl oder Ethyl). Die Durchführung der Reaktion erfolgt in an sich bekannter Weise (vgl. z.B. J. Am. Chem. Soc. 83 (1961) 1733). Die Ausgangsstoffe werden üblicherweise in einem stöchiometrischen Verhältnis eingesetzt. Ein Überschuß bis zu 10% (Gew.%) an einem der beiden Reaktionsteilnehmer über die stöchiometrische Menge hinaus ist möglich. Die Umsetzung wird zweckmäßig in einem inerten Lösungs- oder Verdünnungsmittel (z.B. Diethylether, Tetrahydrofuran, Methyl-tert.-Butylether, Ethylenglykoldimethylether, Toluol, Dimethylsulfoxid) in Gegenwart einer äquivalenten Menge einer Base (z.B. Natriumhydrid, Natriumamid, Kalium-tert.-butylat, Natriummethanolat, Butyllithium, Phenyllithium, Natrium-bis-trimethylsilylamid, Natriummethylsulfinylmethyl) durchgeführt. Die Umsetzungen verlaufen gewöhnlich in einem Temperaturbereich von -70°C bis +30°C. Da sie in einigen Fällen unter Wärmeentwicklung verlaufen, kann es von Vorteil sein, Kühlmöglichkeiten vorzusehen.

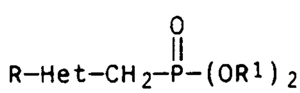

IV

+

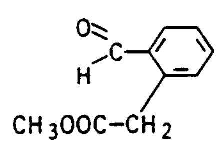

III

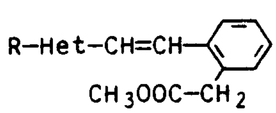

IIa

2-Formyl-phenylessigsäuremethylester III erhält man durch Veresterung von 2-Formyl-phenylessigsäure VII mit Methanol unter Standardbedingungen. Die Darstellung von 2-Formyl-phenylessigsäure VII gelingt in

einfacher Weise durch Ozonolyse des Trimethylsilylenolethers VIII von 2-Indanon IX (Tetrahedron Lett. 25 (1984) 3659; Tetrahedron 43 (1987) 2075).

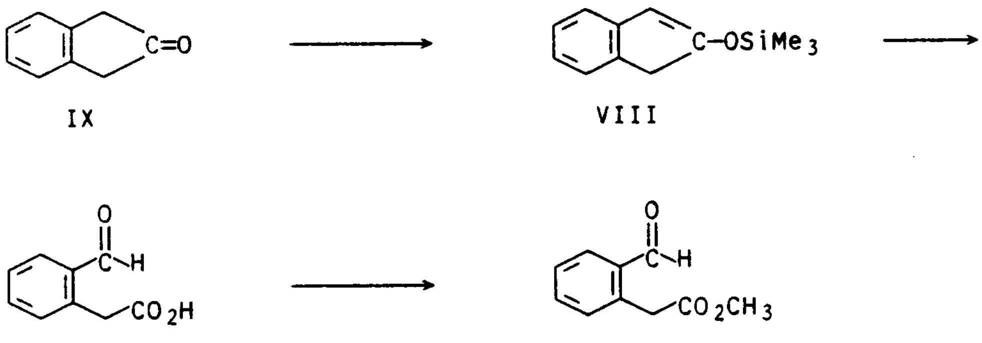

IX VIII VII III

Die heterocyclisch substituierten Methanphosphonsäureester der allgemeinen Formel IV (R und Het haben die oben angegebene Bedeutung, $R^1$ = Methyl, Ethyl) erhält man durch Umsetzung der fünfringheteroaromatischen Methylhalogenverbindungen X der allgemeinen Formel R-Het-$CH_2$-Z (Z = Chlor, Brom) mit Phosphorigsäuretrimethylester oder Phosphorigsäuretriethylester $P(OR^1)_3$ (vgl. Methoden der organischen Chemie, Band 12/1, S. 433, Thieme, Stuttgart 1963).

Zur Herstellung der neuen Verbindungen der allgemeinen Formel I steht noch ein zweites Verfahren zur Verfügung. Dabei werden fünfringheteroaromatische Aldehyde der allgemeinen Formel XII (R und Het haben die oben angegebene Bedeutung) mit 2-(β-Methoxy-α-methoxycarbonyl-vinyl)-benzylphosphonsäuredimethylester XIII umgesetzt (vgl. dazu J. Am. Chem. Soc. 83 (1961) 1733).

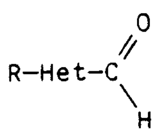

XII

+

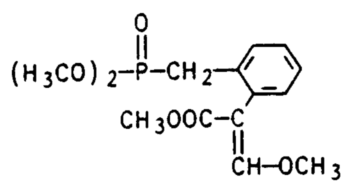

XIII

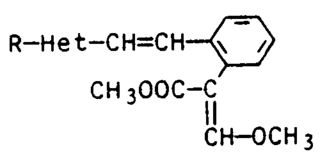

I

2-(β-Methoxy-α-methoxycarbonyl-vinyl)-benzylphosphonsäuredimethylester XIII ist bekannt aus DE-3 519 280 und DE-3 545 318.

Zur Darstellung der neuen Verbindungen der allgemeinen Formel I gemäß Anspruch 1 nach den oben dargestellten Verfahren werden fünfringheteroaromatische Methylhalogenverbindungen X und fünfringheteroaromatische Aldehyde XII als Edukte benötigt. Diese Verbindungen sind entweder bekannt oder können nach bekannten Verfahren hergestellt werden. Entsprechende Herstellverfahren sind z.B. beschrieben in: J. Chem. Soc. (C), 1970, 2563; Synth. Commun. 13 (1983) 741; J. Org. Chem. 50 (1985) 5272; Acta Chem. Scand. 24 (1970) 99; Acta Chem. Scand. 26 (1972) 1851; J. Chem. Soc. 1961, 2733; Liebigs Ann. Chem. 1985, 1377; J. Heterocyclic Chem. 23 (1986) 1535; Synthesis 1982, 318; Eur. J. Med. Chem. 19 (1984) 285; Chem. Pharm. Bull. 34 (1986) 2840; Liebigs Ann. Chem. 717 (1968) 148; Heterocycles 26 (1987) 947; Tetrahedron 43 (1987) 235; J. Chem. Soc., Perkin Trans. I, 1976, 570, Chem. Ber. 106 (1973) 3345; J. Org. Chem. 43 (1978) 3736; J. Org. Chem. 43 (1978) 3742; J. Indian Chem. Soc. 64 (1987) 314; Chem. Ber. 121 (1988) 723; DE-3118258; Chem. Ber. 101 (1968) 3872.

Die neuen Verbindungen der allgemeinen Formel I gemäß Anspruch 1 können als E- oder Z-Isomere an den Doppelbindungen (β-Methoxy-acrylsäuremethylestergruppe und Seitenkette für A = Ethenylen) vorliegen. Die Stereoisomeren lassen sich beispielsweise durch Säulenchromatographie trennen oder aufgrund von Löslichkeitsunterschieden in reiner Form isolieren. Die isomerenreinen Verbindungen lassen sich durch bekannte Methoden in die jeweils anderen Isomeren überführen. Die isomerenreinen Verbindungen werden

wie ihre Mischungen von der vorliegenden Erfindung erfaßt. Für die Anwendung der neuen Verbindungen als Fungizide und Insektizide sind sowohl die Diastereomerengemische als auch die einheitlichen isomeren Verbindungen wie auch deren bei der Synthese anfallenden Gemische geeignet.

Die nachstehenden Beispiele erläutern die Synthese der neuen Verbindungen.

Beispiel 1

Alpha-2-[2'-(3"-Cyclopropyl-isoxazol-5"-yl)-ethen-1'-yl]-phenyl-β-methoxy-acrylsäuremethylester

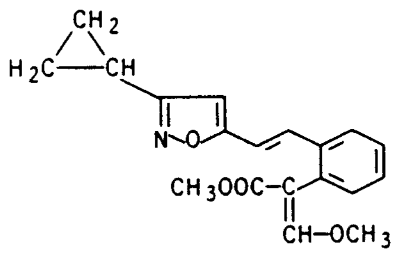

a) Zu einer Lösung von 36,5 g (0.28 mol) 2-Indanon und 32,4 g (0.30 mol) Trimethylchlorsilan in 300 ml Tetrahydrofuran werden bei Raumtemperatur unter Rühren 30,3 g (0.30 mol) Triethylamin gegeben. Man rührt noch 3 Stunden bei Raumtemperatur (20 °C), saugt den ausgefallenen Niederschlag ab und engt das Filtrat ein. Der Rückstand wird durch Destillation (53 °C, 0,3 mbar) gereinigt. Man erhält so 40,6 g (72 %) 2-Trimethylsilyloxy-1H-Inden als farblose Flüssigkeit.

b) 40,0 g (0.20 mol) 2-Trimethylsilyloxy-1H-Inden werden in einer Mischung aus 500 ml Methanol und 150 ml Methylenchlorid gelöst und innerhalb von 5 Stunden bei -70 °C mit 14,0 g (0.30 mol) Ozon versetzt. Nach dem Entfernen von überschüssigem Ozon mit Stickstoff werden 150 ml (2.0 mol) Dimethylsulfid zugegeben und über Nacht bei Raumtemperatur gerührt. Danach wird die Lösung eingeengt und der Rückstand in $NaHCO_3$-Lösung aufgenommen. Die wäßrige Phase wird mit Diethylether gewaschen und mit HCl (verd.) auf pH 2 gebracht. Danach wird mit Diethylether extrahiert, die vereinigten organischen Phasen über $MgSO_4$ getrocknet und eingeengt. Man erhält 24,1 g (75 %) 2-Formyl-phenylessigsäure in Form farbloser Kristalle (Fp.: 103-105 °C).

c) 24,0 g (0.15 mol) 2-Formyl-phenylessigsäure und 0,1 g p-Toluolsulfonsäure werden in 250 ml Methanol 2 Stunden am Rückfluß gekocht. Anschließend wird die Lösung eingeengt, der Rückstand in Diethylether aufgenommen und mit verdünnter HCl gewaschen. Die organische Phase wird abgetrennt, über $MgSO_4$ getrocknet und eingeengt. Der Rückstand wird durch Destillation (90 °C, 0,4 mbar) gereinigt. Man erhält so 19,3 g (74 %) 2-Formylphenylessigsäuremethylester als farblose Flüssigkeit.

d) Zu einer Lösung von 15,6 g (0.06 mol) 3-Cyclopropyl-isoxazol-5-yl-methanphosphonsäurediethylester in 50 ml Tetrahydrofuran werden bei 20 °C 43,3 ml einer 1,5-molaren Lösung von n-Butyllithium in Hexan (0.065 mmol) zugetropft. Man rührt noch 20 Minuten bei 20 °C und tropft dann bei dieser Temperatur eine Lösung von 10,7 g (0.06 mol) 2-Formylphenylessigsäuremethylester in Tetrahydrofuran zu. Die Reaktionsmischung wird über Nacht nachgerührt, auf Eiswasser gegossen und mit Methyl-tert.-Butylether extrahiert. Die organischen Phasen werden mit Wasser gewaschen, über $MgSO_4$ getrocknet und eingeengt. Der Rückstand wird an Kieselgel (Cyclohexan/Essigester = 8/2) chromatographiert. Man erhält 8,0 g (47 %) 2-[2'-(3"-Cyclopropyl-isoxazol-5"-yl)-ethen-1'-yl]-phenylessigsäuremethylester als farbloses Öl.

$^1$H-NMR ($CDCl_3$): 0,87 (m, 2H); 1,03 (m, 2H); 3,68 (s, 3H); 3,77 (s, 2H); 5,93 (s, 1H); 6,80 (d, 1H); 7,25-7,62 (m, 4H); 7,52 (d, 1H).

e) Zu einer Suspension aus 0,76 g (0.03 mol) Natriumhydrid in 30 ml Diethylether wird bei Raumtemperatur eine Mischung aus 6,0 g (0.02 mol) 2-[2'-(3"-Cyclopropyl-isoxazol-5"-yl)-ethen-1'-yl]-phenylessigsäuremethylester, 2,8 g (0.05 mol) Ameisensäuremethylester und 50 ml Diethylether getropft. Man rührt 12 Stunden bei Raumtemperatur und hydrolysiert anschließend mit Eiswasser. Die wäßrige Phase wird mit HCl (verd.) auf pH 4 eingestellt und mit Diethylether extrahiert. Die vereinigten Etherphasen werden über $MgSO_4$ getrocknet und eingeengt. Man erhält 5,4 g (82 %) Alpha-2-[2'-(3"-Cyclopropyl-isoxazol-5"-yl)-ethen-1'-yl]-phenyl-β-hydroxy-acrylsäuremethylester als farbloses Öl.

f) 5,4 g (0.02 mol) des unter e) erhaltenen Acrylsäuremethylesters, 2,4 g (0.02 mol) Kaliumcarbonat und 2,2 g (0.02 mol) Dimethylsulfat werden in 60 ml Aceton 12 Stunden bei Raumtemperatur gerührt.

Anschließend wird vom Niederschlag abfiltriert, das Filtrat eingeengt und der Rückstand in Diethylether aufgenommen. Die organische Phase wird mit verdünnter $NH_4OH$-Lösung gewaschen, über $MgSO_4$ getrocknet und eingeengt. Der Rückstand wird durch Chromatographie an Kieselgel (Cyclohexan/Essigester = 8/2) gereinigt. Das erhaltene Öl wird mit Diisopropylether überschichtet und durch Anreiben kristallisiert. Man erhält 4,5 g (80 %) α-2-[2′-(3″-Cyclopropyl-isoxazol-5″-yl)-ethen-1′-yl]-phenyl-β-methoxy-acrylsäuremethylester in Form farbloser Kristalle (Fp.: 109-111 °C, Verbindung Nr. 42).

Beispiel 2

Alpha-2-[2'-(N-para-Chlorphenyl-pyrrol-3''-yl)-ethen-1'-yl]-phenyl-β-methoxy-acrylsäuremethylester

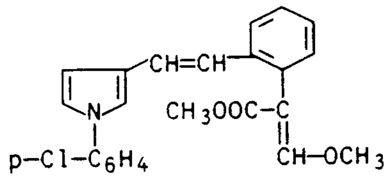

Zu einer Suspension von 1,1 g (0.04 mol) Natriumhydrid in 50 ml Dimethylformamid wird bei 0 °C eine Lösung von 13,7 g (0.04 mol) 2-(β-Methoxy-α-methoxycarbonyl-vinyl)-benzylphosphonsäuredimethylester und 9,0 g (0.04 mol) N-para-Chlorphenyl-pyrrol-3-yl-carboxaldehyd in 100 ml Dimethylformamid unter Rühren getropft. Man rührt noch 1 Stunde bei 0 °C und 12 Stunden bei Raumtemperatur. Anschließend wird mit Eiswasser hydrolysiert und mit Diethylether extrahiert. Die organische Phase wird über $MgSO_4$ getrocknet. Beim Einengen der Etherphase setzt die Kristallisation ein. Man erhält so 5,4 g (31 %) der Titelverbindung in Form farbloser Kristalle (Fp.: 146-147 °C, Verbindung Nr. 4).

In entsprechender Weise lassen sich die folgenden Verbindungen herstellen:

Tabelle 1

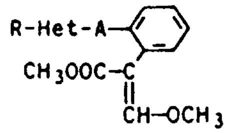

(I)

Verbindungen der allgemeinen Formel I.

Die erste Konfigurationsangabe bezieht sich auf die β-Methoxy-acrylsäuremethylestergruppe, die zweite auf die Ethenylengruppe in den Verbindungen der allgemeinen Formel I.

| Nr. | R | Position von R an Het | Het-A- | Fp.: (°C) (Isomeres) |
|---|---|---|---|---|
| 1 | $C_6H_5$ | 1 | 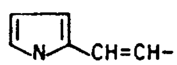 | |
| 2 | $4-Cl-C_6H_4$ | 1 | 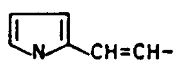 | |
| 3 | $C_6H_5$ | 1 | 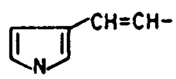 | 133-135 (E,E) |
| 4 | $4-Cl-C_6H_4$ | 1 | 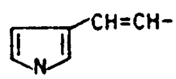 | 146-147 (E,E) |
| 5 | $4-Br-C_6H_4$ | 1 | 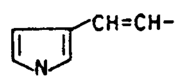 | |
| 6 | $4-OCH_3-C_6H_4$ | 1 | 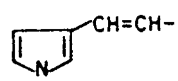 | |

Tabelle 1 (Fortsetzung)

| Nr. | R | Position von R an Het | Het-A- | Fp.: (°C) (Isomeres) |
|---|---|---|---|---|
| 7 | $4-NO_2-C_6H_4$ | 1 | 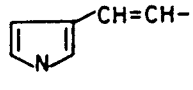 | 142-143 (E,E) |
| 8 | $2,6-(CH_3)_2-C_6H_3$ | 1 | 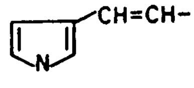 | |

Tabelle 1 (Fortsetzung)

| Nr. | R | Position von R an Het | Het-A- | Fp.: (°C) (Isomeres) |
|---|---|---|---|---|
| 9 | $(CH_3)_2CH$ | 5 | 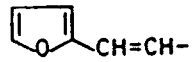 | |
| 10 | cyclo-$C_3H_5$ | 5 | 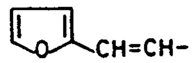 | |
| 11 | 4-Cl-$C_6H_4$ | 5 | 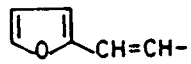 | |
| 12 | 4-$OCH_3$-$C_6H_4$ | 5 | 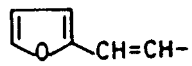 | |

Tabelle 1 (Fortsetzung)

| Nr. | R | Position von R an Het | Het-A- | Fp.: (°C) (Isomeres) |
|---|---|---|---|---|
| 13 | $(CH_3)_2CH$ | 4 | 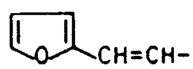 | |
| 14 | cyclo-$C_3H_5$ | 4 | 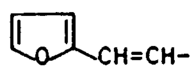 | |
| 15 | 4-Cl-$C_6H_4$ | 4 | 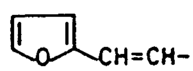 | |
| 16 | 4-$OCH_3$-$C_6H_4$ | 4 | 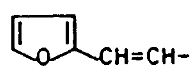 | |
| 17 | $(CH_3)_2CH$ | 5 | 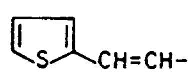 | |

Tabelle 1 (Fortsetzung)

| Nr. | R | Position von R an Het | Het-A- | Fp.: (°C) (Isomeres) |
|---|---|---|---|---|
| 18 | cyclo-$C_3H_5$ | 5 | 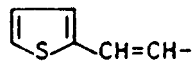 | |
| 19 | 4-Cl-$C_6H_4$ | 5 | 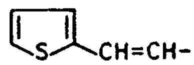 | |
| 20 | 4-$OCH_3$-$C_6H_4$ | 5 | 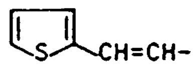 | |
| 21 | $(CH_3)_2CH$ | 4 | 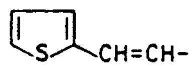 | |
| 22 | cyclo-$C_3H_5$ | 4 | 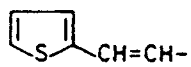 | |

Tabelle 1 (Fortsetzung)

| Nr. | R | Position von R an Het | Het-A- | Fp.: (°C) (Isomeres) |
|---|---|---|---|---|
| 23 | 4-Cl-$C_6H_4$ | 4 | 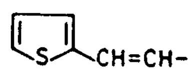 | |
| 24 | 4-$OCH_3$-$C_6H_4$ | 4 | 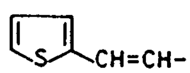 | |
| 25 | $(CH_3)_2CH$ | 5 | 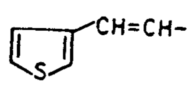 | |
| 26 | cyclo-$C_3H_5$ | 5 | 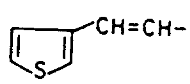 | |
| 27 | 4-Cl-$C_6H_4$ | 5 | 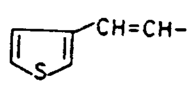 | |

Tabelle 1 (Fortsetzung)

| Nr. | R | Position von R an Het | Het-A- | Fp.: (°C) (Isomeres) |
|---|---|---|---|---|
| 28 | 4-$OCH_3$-$C_6H_4$ | 5 | 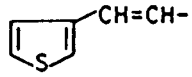 | |
| 29 | $C_6H_5$ | 1 | 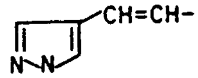 | |
| 30 | 4-$CH_3$-$C_6H_4$ | 1 | 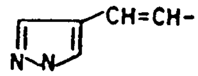 | 120-122 (E,E) |
| 31 | 4-Cl-$C_6H_4$ | 1 | 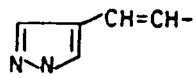 | 141-143 (E,E) |
| 32 | 4-$OCH_3$-$C_6H_4$ | 1 | 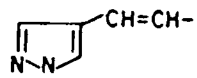 | |

Tabelle 1 (Fortsetzung)

| Nr. | R | Position von R an Het | Het-A- | Fp.: (°C) (Isomeres) |
|---|---|---|---|---|
| 33 | $C_6H_5$ | 5 | 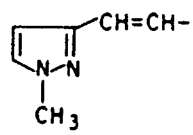 | |
| 34 | cyclo-$C_3H_5$ | 5 | 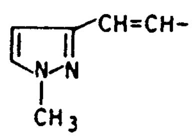 | |
| 35 | 4-Cl-$C_6H_4$ | 5 | 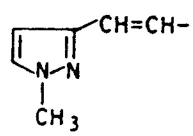 | |

Tabelle 1 (Fortsetzung)

| Nr. | R | Position von R an Het | Het-A- | Fp.: (°C) (Isomeres) |
| --- | --- | --- | --- | --- |
| 36 | 4-$OCH_3$-$C_6H_4$ | 5 | 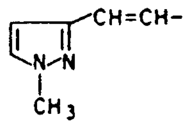 | |

Tabelle 1 (Fortsetzung)

| Nr. | R | Position von R an Het | Het-A- | Fp.: (°C) (Isomeres) |
| --- | --- | --- | --- | --- |
| 37 | $C_6H_5$ | 1 | 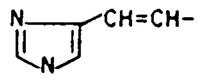 | |
| 38 | 4-$CH_3$-$C_6H_4$ | 1 | 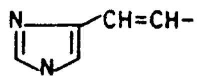 | |
| 39 | 4-Cl-$C_6H_4$ | 1 | 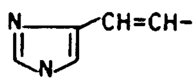 | |
| 40 | 4-$OCH_3$-$C_6H_4$ | 1 | 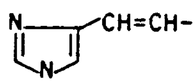 | |
| 41 | $(CH_3)_2CH$ | 3 | 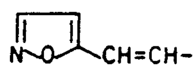 | 90- 91 (E,E) |

Tabelle 1 (Fortsetzung)

| Nr. | R | Position von R an Het | Het-A- | Fp.: (°C) (Isomeres) |
|---|---|---|---|---|
| 42 | cyclo-$C_3H_5$ | 3 | 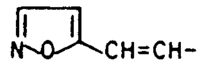 | 109-111 (E, E) |
| 43 | 4-Cl-$C_6H_4$ | 3 | 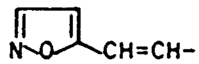 | 161-163 (E, E) |
| 44 | 4-$OCH_3$-$C_6H_4$ | 3 | 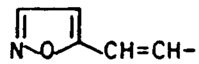 | 130-132 (E, E) |
| 45 | 4-$CH_3$-$C_6H_4$ | 3 | 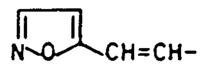 | 138-140 (E, E) |
| 46 | 4-CN-$C_6H_4$ | 3 | 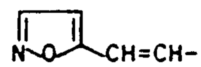 | 163-165 (E, E) |
| 47 | 2,6-$F_2$-$C_6H_3$ | 3 | 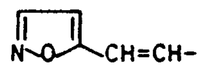 | 126-128 (E, E) |
| 48 | $CO_2C_2H_5$ | 3 | 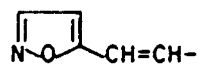 | |

Tabelle 1 (Fortsetzung)

| Nr. | R | Position von R an Het | Het-A- | Fp.: (°C) (Isomeres) |
|---|---|---|---|---|
| 49 | $(CH_3)_2CH$ | 5 | 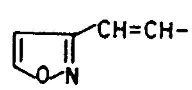 | |
| 50 | cyclo-$C_3H_5$ | 5 | 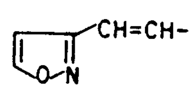 | |
| 51 | 4-Cl-$C_6H_4$ | 5 | 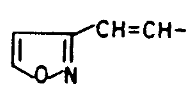 | |

Tabelle 1 (Fortsetzung)

| Nr. | R | Position von R an Het | Het-A- | Fp.: (°C) (Isomeres) |
|---|---|---|---|---|
| 52 | 4-$OCH_3$-$C_6H_4$ | 5 | 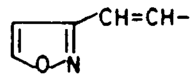 | |
| 53 | $(CH_3)_2CH$ | 3 | 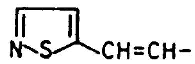 | |
| 54 | cyclo-$C_3H_5$ | 3 | 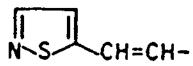 | |
| 55 | 4-Cl-$C_6H_4$ | 3 | 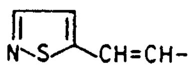 | |
| 56 | 4-$OCH_3$-$C_6H_4$ | 3 | 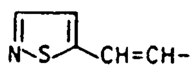 | |

Tabelle 1 (Fortsetzung)

| Nr. | R | Position von R an Het | Het-A- | Fp.: (°C) (Isomeres) |
|---|---|---|---|---|
| 57 | $(CH_3)_2CH$ | 2 | 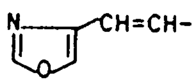 | |
| 58 | cyclo-$C_3H_5$ | 2 | 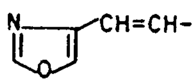 | |
| 59 | 4-Cl-$C_6H_4$ | 2 | 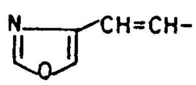 | |
| 60 | 4-$OCH_3$-$C_6H_4$ | 2 | 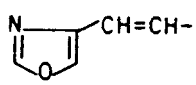 | |

Tabelle 1 (Fortsetzung)

| Nr. | R | Position von R an Het | Het-A- | Fp.: (°C) (Isomeres) |
|---|---|---|---|---|
| 61 | $(CH_3)_2CH$ | 2 | 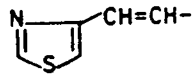 | |
| 62 | cyclo-$C_3H_5$ | 2 | 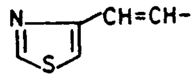 | |
| 63 | 4-Cl-$C_6H_4$ | 2 | 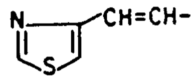 | |
| 64 | 4-$OCH_3$-$C_6H_4$ | 2 | 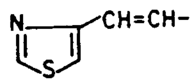 | |

Tabelle 1 (Fortsetzung)

| Nr. | R | Position von R an Het | Het-A- | Fp.: (°C) (Isomeres) |
|---|---|---|---|---|
| 65 | $(CH_3)_2CH$ | 3 | 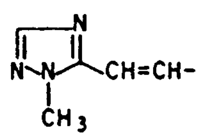 | |
| 66 | cyclo-$C_3H_5$ | 3 | 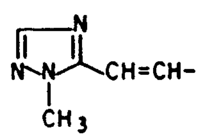 | |
| 67 | 4-Cl-$C_6H_4$ | 3 | 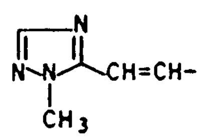 | |
| 68 | 4-$OCH_3$-$C_6H_4$ | 3 | 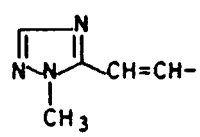 | |

Tabelle 1 (Fortsetzung)

| Nr. | R | Position von R an Het | Het-A- | Fp.: (°C) (Isomeres) |
|---|---|---|---|---|
| 69 | $(CH_3)_2CH$ | 5 | 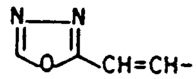 | 91- 92 (E,E) |
| 70 | cyclo-$C_3H_5$ | 5 | 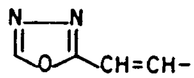 | |
| 71 | 4-Cl-$C_6H_4$ | 5 | 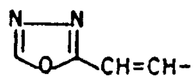 | |

Tabelle 1 (Fortsetzung)

| Nr. | R | Position von R an Het | Het-A- | Fp.: (°C) (Isomeres) |
|---|---|---|---|---|
| 72 | 4-$OCH_3$-$C_6H_4$ | 5 | 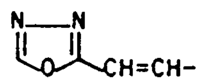 | |
| 73 | $(CH_3)_2CH$ | 5 | 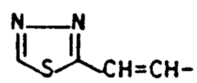 | |
| 74 | cyclo-$C_3H_5$ | 5 | 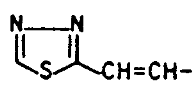 | |
| 75 | $C_2H_5O$ | 5 | 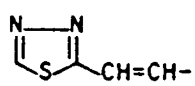 | |
| 76 | 4-Cl-$C_6H_4$ | 5 | 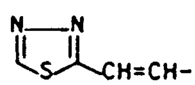 | 152-153 (E,E) |

Tabelle 1 (Fortsetzung)

| Nr. | R | Position von R an Het | Het-A- | Fp.: (°C) (Isomeres) |
|---|---|---|---|---|
| 77 | 4-$OCH_3$-$C_6H_4$ | 5 | 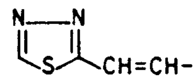 | |
| 78 | $(CH_3)_2CH$ | 5 | 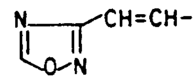 | |
| 79 | cyclo-$C_3H_5$ | 5 | 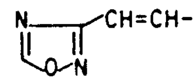 | |

Tabelle 1 (Fortsetzung)

| Nr. | R | Position von R an Het | Het-A- | Fp.: (°C) (Isomeres) |
|---|---|---|---|---|
| 80 | 4-Cl-$C_6H_4$ | 5 | 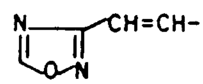 | |
| 81 | 4-$OCH_3$-$C_6H_4$ | 5 | 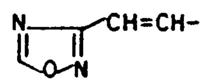 | |
| 82 | $(CH_3)_2CH$ | 3 | 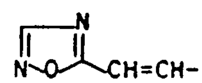 | |
| 83 | cyclo-$C_3H_5$ | 3 | 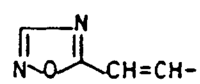 | |
| 84 | 4-Cl-$C_6H_4$ | 3 | 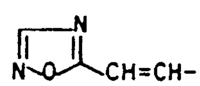 | 111-113 (E,E) |

Tabelle 1 (Fortsetzung)

| Nr. | R | Position von R an Het | Het-A- | Fp.: (°C) (Isomeres) |
|---|---|---|---|---|
| 85 | 4-$OCH_3$-$C_6H_4$ | 3 | 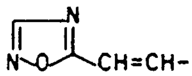 | |
| 86 | $(CH_3)_2CH$ | 3 | 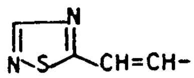 | |
| 87 | cyclo-$C_3H_5$ | 3 | 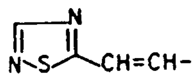 | |
| 88 | 4-Cl-$C_6H_4$ | 3 | 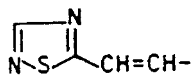 | |
| 89 | 4-$OCH_3$-$C_6H_4$ | 3 | 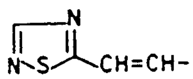 | |

Tabelle 1 (Fortsetzung)

| Nr. | R | Position von R an Het | Het-A- | Fp.: (°C) (Isomeres) |
|---|---|---|---|---|
| 90 | 2-F-$C_6H_4$ | 1 | 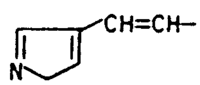 | 91- 92 (E,E) |
| 91 | 3-F-$C_6H_4$ | 1 | 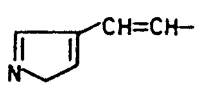 | 138-139 (E,E) |
| 92 | 4-F-$C_6H_4$ | 1 | 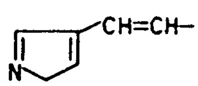 | 138 (E,E) |
| 93 | 3-Cl-$C_6H_4$ | 1 | 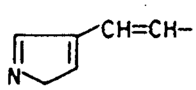 | 141-142 (E,E) |
| 94 | 4-$CH_3$-$C_6H_4$ | 1 | 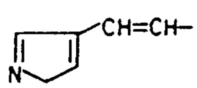 | 124-125 (E,E) |

Tabelle 1 (Fortsetzung)

| Nr. | R | Position von R an Het | Het-A- | Fp.: (°C) (Isomeres) |
|---|---|---|---|---|
| 95 | $4\text{-}t\text{-}C_4H_9\text{-}C_6H_4$ | 1 | 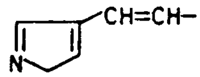 | 105-106 (E,E) |
| 96 | $2,6\text{-}F_2\text{-}C_6H_3$ | 1 | 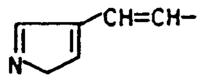 | 116-117 (E,E) |
| 97 | $3,4\text{-}Cl_2\text{-}C_6H_3$ | 1 | 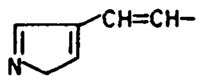 | 147-149 (E,E) |
| 98 | $4\text{-}F\text{-}C_6H_4$ | 1 | 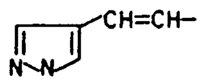 | 115-117 (E,E) |
| 99 | $4\text{-}Br\text{-}C_6H_4$ | 1 | 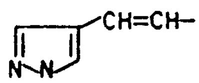 | 138-140 (E,E) |
| 100 | $CH_3$ | 3 | 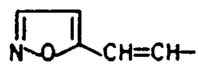 | 155-157 (E,E) |
| 101 | $C_6H_5$ | 3 | 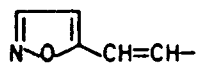 | 117-119 (E,E) |
| 102 | $2\text{-}Cl\text{-}6\text{-}F\text{-}C_6H_3$ | 3 | 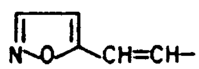 | Öl (E,E) |
| 103 | $4\text{-}CH_3\text{-}C_6H_4$ | 5 | 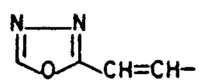 | 147 (E,E) |
| 104 | $C_6H_5$ | 5 | 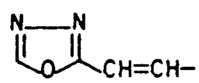 | 144 (E,E) |

Tabelle 2

NMR-Daten ausgewählter Verbindungen aus der Tabelle 1. Die chemische Verschiebung (δ) wird in ppm relativ zu Tetramethylsilan angegeben. Als Lösungsmittel dient $CDCl_3$.

Verbindung Nr. 4
3.70 (s, 3H); 3. 80 (s, 3H); 6.53 (d, 1H); 6.80 (d, 1H); 6.95 (d, 1H); 7.00 (d, 1H); 7.10 (s, 1H); 7.15 - 7.70 (m, 8H); 7.63 (s, 1H).

Verbindung Nr. 42
0.82 (m, 2H); 1.00 (m, 2H); 2.00 (m, 1H); 3.68 (s, 3H); 3.80 (s, 3H); 5.88 (s, 1H); 6.84 (d, 1H); 7.17 - 7.68 (m, 4H); 7.28 (d, 1H); 7.65 (s, 1H).

Verbindung Nr. 43
3.70 (s, 3H); 3.82 (s, 3H); 6.50 (s, 1H); 6.92 (d, 1H); 7.19-7.79 (m, 9H); 7.68 (s, 1H).

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten fungiziden Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 4 mit 10 Gew.-Teilen N-Methyl-$\alpha$-pyrrolidon und erhält eine Lösung, die zur Anwendung in Form kleinster Tropfen geeignet ist.

III. 20 Gew.-Teile der Verbindung Nr. 42 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 4 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

VI. 3 Gew.-Teile der Verbindung Nr. 42 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 4 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

IX. 20 Gew.-Teile der Verbindung Nr. 42 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Anwendungsbeispiele

Als Vergleichswirkstoff wurde die Verbindung $\alpha$-2-(6-Chlorpyrazin-2-oxy)-phenyl-$\beta$-methoxy-acrylsäuremethylester (C) - bekannt aus EP-260 794 - verwendet.

Anwendungsbeispiel 1

Wirksamkeit gegen Plasmopara viticola

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24 °C und anschließend für 5 Tage in einem Gewächshaus mit Temperaturen

zwischen 20 und 30°C aufgestellt. Nach dieser Zeit wurden die Pflanzen zur Beschleunigung des Sporangienträgerausbruches abermals für 16 Stunden in der feuchten Kammer aufgestellt. Dann erfolgte die Beurteilung des Ausmaßes des Pilzausbruches auf den Blattunterseiten.

Das Ergebnis des Versuches zeigt, daß die Wirkstoffe 30, 31, 42, 43, 44, 45, 46, 76, 92, 98, 99, 100, 101 und 102 bei der Anwendung als 0,0125 gew.%ige Spritzbrühe eine bessere fungizide Wirkung zeigen (95 %) als der bekannte Vergleichswirkstoff C (60 %).

Anwendungsbeispiel 2

Wirksamkeit gegen Septoria nodorum

Weizenpflanzen der Sorte "Frühgold" wurden im Ein-Blatt-Stadium mit wäßriger Wirkstoffaufbereitung, die 80 % Wirkstoff und 20 % Emulgiermittel in der Trockensubstanz enthielt, bis zur Tropfnässe besprüht. 20 Stunden nach dem Antrocknen des Spritzbelages wurden die Pflanzen mit einer wäßrigen Sporensuspension von Septoria nodorum bis zur Tröpfchenbildung inokuliert und für eine Woche in eine Klimakammer bei Temperaturen zwischen 17 und 19°C und einer relativen Luftfeuchtigkeit von ungefähr 90 bis 95 % gestellt. Dann wurde das Ausmaß der Symptomentwicklung ermittelt.

Das Ergebnis des Versuches zeigt, daß die Wirkstoffe 4, 30, 31, 41, 69, 84, 90, 91, 94, 98, 99, 102 und 103 bei der Anwendung als 0,05 gew.%ige Spritzbrühe eine sehr gute fungizide Wirkung haben (95 %).

Anwendungsbeispiel 3

Wirksamkeit gegen Pyrenophora teres

Gerstenkeimlinge der Sorte "Igri" wurden im Zweiblattstadium mit wäßrigen Suspensionen, die 80 % Wirkstoff und 20 % Emulgator in der Trockensubstanz enthielten, tropfnaß gespritzt. Nach 24 Stunden wurden die Pflanzen mit einer Sporensuspension des Pilzes Pyrenophora teres inokuliert und für 48 Stunden in eine Klimakammer mit hoher Luftfeuchtigkeit bei 18°C gestellt. Anschließend wurden die Pflanzen im Gewächshaus bei 20 bis 22°C und 70 % relativer Luftfeuchtigkeit für weitere 5 Tage kultiviert. Dann wurde das Ausmaß des Befalls ermittelt.

Das Ergebnis des Versuches zeigt, daß die Wirkstoffe 30, 31, 45, 46, 69, 84, 98, 99, 100 und 102 bei der Anwendung als 0,0125 gew.%ige Spritzbrühe eine bessere fungizide Wirkung haben (95 %) als der bekannte Wirkstoff C (55 %).

Die neuen Verbindungen sind außerdem geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grndiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Zur Klasse der Nematoden zählen beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Hetrodera glycinae, Heterodera schatii, Hetrodera triflolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Paratylenchus curvitatus, Partylenchus goodeyi.

Für die Anwendung zur Schädlingsbekämpfung können die Wirkstoffkonzentrationen in den anwendungsfertigen Zubereitungen in größeren Bereichen variiert werden.

Im allgemeinen liegen sie zwischen 0,0001 und 10 %, vorzugsweise zwischen 0,001 und 0,1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff für die Schädlingsbekämpfung beträgt unter Freilandbedingungen 0,01 bis 10, vorzugsweise 0,1 bis 1,0 kg/ha.

Als Vergleichswirkstoffe wurden die Verbindung 2-($\beta$-Methoxy-$\alpha$-methoxycarbonylvinyl)-4'-chlor-stilben (A) und $\alpha$-[2-(Benzoxazol-2'-yl-oxy)-phenyl]-$\beta$-methoxy-acrylsäuremethylester (B) - bekannt aus EP 178 826 und EP 256 667 - benutzt.

Anwendungsbeispiel 4

Prodenia litura, ägypt. Baumwollwurm

Versuchart: Fraßwirkung

Versuchsdurchführung:

Die Versuche wurden in 250 ml Plastikbechern durchgeführt. Dabei setzte man 2 Raupen in die Gefäße und bot Stücke von Maispflanzen, die man für 5 Sekunden in die wäßrige Wirkstoffaufbereitung getaucht hatte, als Futter an. Wirkstoffgehalt in ppm. Nach 24 Stunden beurteilte man die Mortalitätsrate in %.

| Wirkstoff-Nr. | Prodenia | |
|---|---|---|
| | ppm | % Mort. |
| 4 | 40 | 100 |
| 43 | 200 | 80 |
| A | 1000 | 0 |
| B | 1000 | 80 |

Anwendungsbeispiel 5

Musca domestica (Stubenfliegen)

Versuchsart: Dauerkontakt

Versuchsdurchführung:

Beide Teile einer Glasschale von 10 cm Durchmesser wurden mit insgesamt 1 ml der acetonischen Lösung der Wirkstoffe benetzt. Wirkstoffgehalt der Lösung in ppm. Nach Verdunsten des Lösungsmittels brachte man je 10 Fliegen in die Schalen, schloß sie und zählte nach 4 Stunden die Tiere, die sich in Rückenlage befanden und bestimmte die Mortalitätsrate in %.

| Wirkstoff-Nr. | Musca | |
|---|---|---|
| | ppm | % Mort. |
| 4 | 2 | 100 |
| A | 2 | 0 |
| B | 4 | 80 |

Anwendungsbeispiel 6

Plutella maculipennis (Kohlschabe)

Versuchsart: Kontaktwirkung

Versuchsdurchführung:

Junge Kohlblätter wurden 3 sec. in die wäßrige Aufbereitung der Prüfsubstanz getaucht (Wirkstoffgehalt in ppm) und auf einen mit 0,5 ml Wasser angefeuchteten Rundfilter (Φ 9 cm) in eine Glasschale gelegt (Φ 10 cm). Darauf wurde das Blatt mit 10 Raupen im 4. Larvenstadium besetzt und die Glasschale geschlossen. Nach 48 Stunden beurteilte man die Mortalität in %.

| Wirkstoff-Nr. | Plutella | |
|---|---|---|
| | ppm | % Mort. |
| 4 | 100 | 100 |
| 43 | 200 | 100 |
| A | 1000 | 0 |
| B | 200 | 0 |
| | 1000 | 80 |

Anwendungsbeispiel 7

Ornithodorus moubata (Zecke)

Versuchsart: Kontaktwirkung

Versuchsdurchführung:

Verwendet wurden junge Zecken, die eine Blutmahlzeit hinter sich hatten (Durchmesser 1,5 bis 2 mm). Diese Tiere nahm man mit Hilfe eines Saugrohres einzeln auf, wobei eine starke Lichtquelle die aktiven Tiere aus den Häutungsresten trieb. Je 5 Zecken wurden in einem Papierbeutel für 5 Sekunden in die wäßrige Wirkstoffaufbereitung getaucht (Wirkstoffgehalt in ppm). Die Beutel hing man danach frei auf und ermittelte nach 48 Stunden die Wirkung. Dabei wurden die Beutel an eine starke Lichtquele (60 W-Birne) gehalten; die lebenden Tiere versuchten auszuweichen und waren leicht zu erkennen. Die Versuchstemperatur betrug ca. 25 ° C. Man bestimmte die Mortalität in %.

| Wirkstoff-Nr. | Zecken | |
|---|---|---|
| | ppm | % Mort. |
| 4 | 400 | 80 |
| 41 | 1000 | 80 |
| 45 | 1000 | 60 |
| 101 | 1000 | 60 |
| A | 1000 | 0 |
| B | 1000 | 0 |

Anwendungsbeispiel 8

Tetranychus telarius, Rote Spinne, Kontaktwirkung, Spritzversuch

Buschbohnen in Töpfen, die das zweite Blattpaar zeigen, werden mit der wäßrigen Wirkstoffaufbereitung tropfnaß gespritzt. Die Pflanzen werden von allen Seiten mit insgesamt 50 ml Spritzbrühe besprüht. Die Pflanzen weisen einen starken Milbenbefall und reichliche Eiablage auf.

Die Wirkung wird nach 5 Tagen mittels Binokular ermittelt. Dabei wird darauf geachtet, ob alle Entwicklungsstadien der Tiere gleichmäßig erfaßt sind. Die Pflanzen stehen während der 5 Tage unter normalen Gewächshausbedingungen.

| Wirkstoff-Nr. | ppm | % Mort. |
|---|---|---|
| 41 | 100 | 100 |
| 45 | 40 | 80 |
| 46 | 400 | 100 |
| 101 | 100 | 100 |
| B | 1000 | 0 |

## Patentansprüche

**1.** Heterocyclisch substituierte $\alpha$-Aryl-acrylsäuremethylester der allgemeinen Formel

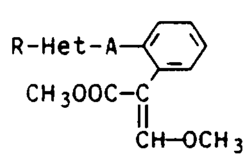

(I)

in der

R $C_1$-$C_8$-Alkyl, $C_2$-$C_8$-Alkenyl, $C_3$-$C_6$-Cycloalkyl, $C_1$-$C_4$-Alkoxy, ($C_1$-$C_4$-Alkyl)carbonyl, ($C_1$-$C_4$-Alkoxy)carbonyl oder Aryl bedeutet, wobei der aromatische Ring gegebenenfalls substituiert ist durch $C_1$-$C_8$-Alkyl, $C_3$-$C_6$-Cycloalkyl, $C_1$-$C_2$-Halogenalkyl, $C_1$-$C_4$-Alkoxy, Halogen, Cyano, Nitro

Het einen gegebenenfalls durch Methyl an einem Stickstoffatom substituierten fünfringheteroaromatischen Rest mit 1 bis 3 Heteroatomen, ausgewählt aus der Gruppe Sauerstoff, Schwefel oder Stickstoff, der über ein C-Atom an A gebunden ist,

A Ethenylen bedeutet.

**2.** Verfahren zur Herstellung von heterocyclisch substituierten Phenylessigsäuremethylestern der allgemeinen Formel IIa,

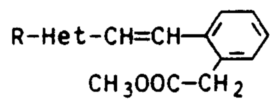

(IIa)

in der

R und Het die im Anspruch 1 genannten Bedeutungen haben,

dadurch gekennzeichnet, daß man einen 2-Formylphenylessigsäuremethylester III mit einem heterocyclisch substituierten Methanphosphonsäureester der allgemeinen Formel IV (R und Het haben die oben angegebene Bedeutung, $R^1$ = Methyl, Ethyl) umsetzt.

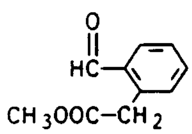

III

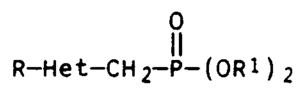

IV

**3.** Substituierte Phenylessigsäuremethylester der Formel

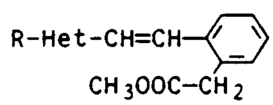

(IIa)

in der

R und Het die im Anspruch 1 genannten Bedeutungen haben.

**4.** Fungizid, enthaltend einen inerten Trägerstoff und eine fungizid wirksame Menge eines heterocyclisch substituierten $\alpha$-Aryl-acrylsäuremethylesters der allgemeinen Formel I gemäß Anspruch 1.

**5.** Verfahren zur Bekämpfung von Pilzen, dadurch gekennzeichnet, daß man die Pilze oder die von Pilzbefall bedrohten Materialien, Pflanzen, Saatgüter oder den Erdboden mit einer fungizid wirksamen Menge eines heterocyclisch substituierten $\alpha$-Aryl-acrylsäuremethylesters der allgemeinen Formel I gemäß Anspruch 1 behandelt.

**6.** Schädlingsbekämpfungsmittel, enthaltend einen inerten Trägerstoff und eine für Schädlinge wirksame Menge eines heterocyclisch substituierten $\alpha$-Aryl-acrylsäuremethylesters der allgemeinen Formel I gemäß Anspruch 1.

**7.** Verfahren zur Bekämpfung von Schädlingen, dadurch gekennzeichnet, daß man eine für Schädlinge wirksame Menge eines heterocyclisch substituierten $\alpha$-Aryl-acrylsäuremethylesters der allgemeinen

Formel I gemäß Anspruch 1 auf die Schädlinge oder deren Lebensraum einwirken läßt.

**8.** Verbindung gemäß Anspruch 1 in der R einen para-Chlorphenylrest in 1-Stellung und Het einen Pyrrol-3-ylrest bedeutet.

**9.** Verbindung gemäß Anspruch 1 in der R einen Cyclopropylrest in 3-Stellung und Het einen Isoxazol-5-ylrest bedeutet.

## Claims

**1.** A methyl α-arylacrylate substituted by a heterocyclic radical and of the general formula

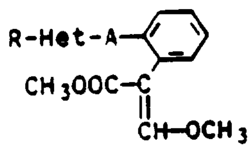

(I)

where R is $C_1$-$C_8$-alkyl, $C_2$-$C_8$-alkenyl, $C_3$-$C_6$-cycloalkyl, $C_1$-$C_4$-alkoxy, ($C_1$-$C_4$-alkyl)carbonyl, ($C_1$-$C_4$-alkoxy)carbonyl or aryl, the aromatic ring being unsubstituted or substituted by $C_1$-$C_8$-alkyl, $C_3$-$C_6$-cycloalkyl, $C_1$- or $C_2$-haloalkyl, $C_1$-$C_4$-alkoxy, halogen, cyano or nitro, Het is a five-membered heteroaromatic ring which is unsubstituted or substituted by methyl at a nitrogen atom and has from 1 to 3 heteroatoms selected from the group consisting of oxygen, sulfur and nitrogen, and is bonded to A via a carbon atom, and A is ethenylene.

**2.** A process for the preparation of a methyl phenylacetate substituted by a heterocyclic radical and of the general formula IIa

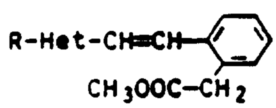

(IIa)

where R and Het have the meanings stated in claim 1, which comprises reacting a methyl 2-formylphenylacetate III with a methanephosphonic ester of the formula IV (where R and Het have the abovementioned meanings and $R^1$ is methyl or ethyl) which is substituted by a heterocyclic radical.

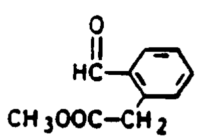

III

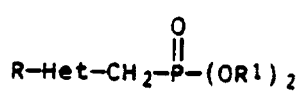

IV

**3.** A substituted methyl phenylacetate of the formula

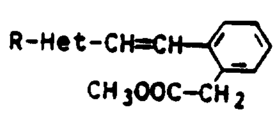

(IIa)

where R and Het have the meanings stated in claim 1.

4. A fungicide containing an inert carrier and a fungicidally effective amount of a methyl α-arylacrylate substituted by a heterocyclic radical and of the general formula I, as claimed in claim 1.

5. A method for controlling fungi, wherein the fungi, or the materials, plants, seed or the soil threatened by fungal attack, are treated with a fungicidally effective amount of a methyl α-arylacrylate substituted by a heterocyclic radical and of the general formula I, as claimed in claim 1.

6. A pesticide containing an inert carrier and a pesticidally effective amount of methyl α-arylacrylate substituted by a heterocyclic radical and of the general formula I, as claimed in claim 1.

7. A method for controlling pests, wherein a pesticidally effective amount of a methyl α-arylacrylate substituted by a heterocyclic radical and of the general formula I, as claimed in claim 1, is allowed to act on the pests or their habitat.

8. A compound as claimed in claim 1, where R is parachlorophenyl in the 1-position and Het is pyrrol-3-yl.

9. A compound as claimed in claim 1, where R is cyclopropyl in the 3-position and Het is isoxazol-5-yl.

**Revendications**

1. α-aryl-acrylates de méthyle à substituants hétérocycliques de formule générale

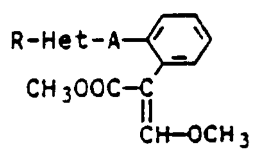

(I)

dans laquelle
R représente un groupe alkyle en C1-C8, alcényle en C2-C8, cycloalkyle en C3-C6, alcoxy en C1-C4, (alkyle en C1-C4)-carbonyle, (alcoxy en C1-C4)-carbonyle ou aryle, le noyau aromatique pouvant éventuellement être substitué par des groupes alkyle en C1-C8, cycloalkyle en C3-C6, halogénoalkyle en C1-C2, alcoxy en C1-C4, des halogènes, des groupes cyano, nitro,
Het représente un radical hétéroaromatique à 5 chaînons éventuellement substitué par un groupe méthyle sur un atome d'azote et contenant de 1 à 3 hétéroatomes choisis parmi l'oxygène, le soufre ou l'azote, et qui est relié à A par l'intermédiaire d'un atome de carbone,
A représente un groupe éthénylène.

2. Procédé de préparation des phénylacétates de méthyle à substituants hétérocycliques de formule générale IIa

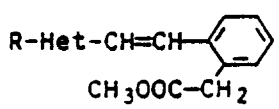

(IIa)

dans laquelle
R et Het ont les significations indiquées dans la revendication 1, caractérisé en ce que l'on fait réagir un 2-formylphénylacétate de méthyle III avec un ester méthane-phosphonique à substituant hétérocyclique de formule générale IV (dans laquelle R et Het ont les significations indiquées ci-dessus et $R^1$ = méthyle, éthyle).

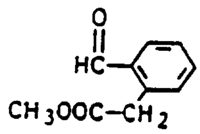 III 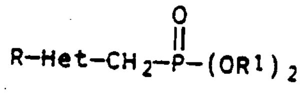 IV

3. Phénylacétates de méthyle substitués de formule

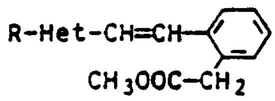 (IIa)

dans laquelle R et Het ont les significations indiquées dans la revendication 1.

4. Produit fongicide contenant un véhicule inerte et une quantité fongicide efficace d'un α-aryl-acrylate de méthyle à substituant hétérocyclique de formule générale I de la revendication 1.

5. Procédé pour combattre les mycètes, caractérisé en ce que l'on traite les mycètes ou les matériaux/végétaux/semences menacés d'une attaque par les mycètes ou le sol par une quantité fongicide efficace d'un α-arylacrylate de méthyle à substituant hétérocyclique de formule générale I de la revendication 1.

6. Produit parasiticide contenant un véhicule inerte et une quantité parasiticide efficace d'un α-aryl-acrylate de méthyle à substituant hétérocyclique de formule générale I de la revendication 1.

7. Procédé pour combattre les parasites, caractérisé en ce que l'on fait réagir sur les parasites ou leur habitat une quantité parasiticide efficace d'un α-aryl-acrylate de méthyle à substituant hétérocyclique de formule générale I de la revendication 1.

8. Composé selon la revendication 1, pour lequel R représente un groupe para-chlorophényle en position 1 et Het représente un groupe pyrrole-3-yle.

9. Composé selon la revendication 1, pour lequel R représente un groupe cyclopropyle en position 3 et Het un groupe isoxazole-5-yle.